# EUROPÄISCHE PATENTSCHRIFT

(11) **EP 0 883 684 B1**
(45) Veröffentlichungstag und Bekanntmachung des Hinweises auf die Patenterteilung: **24.11.2004**
(21) Anmeldenummer: 97903312.3
(22) Anmeldetag: 18.02.1997
(51) Int. Cl.: C12N 9/64, A61K 38/00

(54) **VERFAHREN ZUR REINIGUNG VON THROMBINÄHNLICHEN PROTEASEN AUS SCHLANGENGIFTEN**
METHOD OF PURIFYING THROMBIN-LIKE PROTEASE ENZYMES OBTAINED FROM SNAKE VENOM
PROCEDE POUR LA PURIFICATION DE PROTEASES ANALOGUES A LA THROMBINE ISSUES DU VENIN DE SERPENT

(30) Priorität: 26.02.1996 DE 19607210
(43) Veröffentlichungstag der Anmeldung: 16.12.1998
(73) Patentinhaber: Abbott GmbH & Co. KG, 65205 Wiesbaden (DE)
(72) Erfinder: SCHWARZ, Margarete, D-67146 Deidesheim (DE); ZAHN, Wolfgang, D-67122 Altrip (DE)
(74) Vertreter: Grünecker, Kinkeldey, Stockmair & Schwanhäusser Anwaltssozietät
(86) Internationale Anmeldenummer: PCT/EP1997/000755
(87) Internationale Veröffentlichungsnummer: WO 1997/032015

(56) Entgegenhaltungen:
- EP-A- 0 020 780
- EP-A- 0 328 256
- DE-A- 1 442 134
- DE-A- 2 718 542
- GB-A- 1 177 506
- US-A- 3 743 722
- TOXICON, Bd. 29, Nr. 11, 1991, Seiten 1381-1386, XP000609607 QIAN XIAOHONG ET AL: "FIBRINOLYTIC ENZYME FROM AGKISTRODON HALYS BREVICAUDUS (KOREAN MAMUSHI) SNAKE VENOM"

## Beschreibung

Die vorliegende Erfindung betrifft ein Verfahren zur Reinigung von thrombinähnlichen Proteasen aus Schlangengiften.

Beispiele für solche Proteasen sind Batroxobin, Crotalase und insbesondere Ancrod. Letztere ist ein Antikoagulanz, welches aus dem Gift der Schlange Agkistrodon rhodostoma isoliert wird (Merck-Index 1989, Nr. 664). Zu seiner Herstellung aus Schlangengift sind bereits eine Vielzahl von Methoden beschrieben worden (GB-PS 1.094.301, GB-PS 1.177.506, GB-PS 1.293.793, US-PS 3.743.722, US-PS 3.879.369, DE-OS 2.428.955, DE-OS 2.734.427). Diese Verfahren basieren im wesentlichen auf chromatographischen Schritten und liefern das Ancrod in unterschiedlicher Ausbeute und Reinheit.

Die Herstellung von hochreinem Ancrod aus Schlangengift ist bisher nicht gelungen. Es wurde immer ein Gemisch von Enzymen mit Ancrod als Hauptkomponente isoliert, das je nach Herstellung mit mehr oder weniger Fremdproteinen verunreinigt war.

Es wurde nun ein Weg gefunden, um thrombinähnliche Proteasen aus Schlangengiften in hochreiner Form herzustellen.

Gegenstand der Erfindung ist ein Verfahren zur Reinigung von thrombinähnlichen Proteasen aus Schlangengiften, welches darin besteht, daß man
a. ein Protease-Rohprodukt einer Vorreinigung durch Affinitätschromatographie oder Chromatographie an einem basischen Ionenaustauscher unterwirft,
b. die so erhaltene Fraktion mit den thrombinähnlichen Enzymen einer Chromatographie an einem schwachen Kationenaustauscher unterwirft oder durch Adsorption an Glas im basischen Bereich trennt und
c. die Hauptkomponente aus Schritt b einer Gelchromatograhie unterwirft oder diese durch Chromatographie an Glas im sauren Bereich reinigt,
wobei jedoch mindestens einer der Schritte b und c eine Trennung durch Adsorption bzw. Chromatographie an Glas beinhaltet.

Es empfiehlt sich, für den Schritt b die Reinigung durch Adsorption an Glas und den Schritt c mit Hilfe der Gelchromatograpie durchzuführen.

In einer besonders bevorzugten Ausführungsform der Erfindung wird sowohl in Schritt b als auch in Schritt c durch Adsorption bzw. Chromatographie an Glas gereinigt.

Zur Vorreinigung durch Affinitätschromatographie eignet sich besonders Agmatin-, Arginin- oder Heparin-Sepharose.

Als basische Ionenaustauscher eignen sich für die Vorreinigung besonders DEAE-Cellulose und DEAE-Sepharose.

Als Puffer seien für die Ionenaustauschchromatographie insbesondere Tris-phosphat- und Natriumphosphat-Puffer genannt.

Die Ionenaustauschchromatographie wird bei einem pH-Wert von 5-9, vorzugsweise von 6-8,5 durchgeführt.

Bei der Vorreinigung werden etwa 70-80% an Fremdproteinen und anderen Bestandteilen aus dem Rohenzym entfernt.

Wird der zweite Schritt (b) der Reinigung mit einem Kationenaustauscher durchgeführt, so kommen folgende schwach saure Austauscher in Frage: CM-SEPHAROSE, pH-Wert 5-9, und AMBERLITE CG50, pH 5-9.

Chromatographie an Glas bedeutet, daß Ancrod und verwandte thrombinähnliche Enzyme sowie stark basische Proteasen bei pH-Werten von 7,5-9,0, vorzugsweise 8,0-8,5 an die Glas-Matrix gebunden werden. Ca. 60 % an vor allem sauren Fremdproteinen werden ungebunden mit dem Equilibrierungspuffer (vorzugsweise Tris-Phosphat- bzw. Natrium-Phosphat-Puffer) von der Säule gewaschen. Ancrod wird in über 90%iger Reinheit fraktioniert vom Glas eluiert, indem die Ionenstärke des Puffers durch Zugabe von Kochsalz auf 0,3-1,0 M erhöht wird.

Im zweiten Reinigungsschritt (b) wird das Enzym bis auf etwa 90 % angereichert.

Für die Gelchromatographie als Reinigungsschritt c kommen insbesondere in Frage: SEPHACRYL S-100HR, SUPERDEX, SEPHADEX, ULTRO-GEL und SUPEROSE.

Wählt man für diesen Reinigungsschritt c die Chromatographie an Glas, so werden im sauren pH-Bereich von 4-6 basische Fremdproteine aus der Ancrodlösung an der Glasoberfläche adsorbiert, während sich Ancrod in weit über 95%iger Reinheit direkt im Equilibrierungspuffer von der Säule eluieren läßt. Die gewünschte Ionenstärke des Puffers kann durch Zugabe eines Salzes wie Kochsalz reguliert werden.

Das neue Verfahren eignet sich ganz besonders zur Reindarstellung von Ancrod, welches nach diesem Reinigungsverfahren in einer Reinheit von deutlich über 95% anfällt.

### Beispiel 1

### a. Vorreinigung

3 g getrocknetes Gift der malaiischen Grubenotter wurden in 50 ml Tris-(hydroxymethyl)-aminomethan (TRIS)-Phosphat-Puffer pH 8,5 gelöst, unlösliche Zellbestandteile des Giftes abzentrifugiert und die klare, gelbe Lösung auf eine Chromatographie-Säule aufgetragen, welche einen Durchmesser von 1,6 cm aufwies und bis zu einer Höhe von 30 cm mit DEAE-SEPHAROSE-FF (Firma Pharmacia) gefüllt war. Die thrombinähnlichen Enzyme des Giftes sowie die Proteine mit saurem Charakter wurden an die Matrix gebunden. Die Chromatographie wurde mit einer Durchflußgeschwindigkeit von 150 - 200 ml/h durchgeführt. Durch Waschen der Säule bei Raumtemperatur mit ca. 300 ml Equilibrierungspuffer (10 mM TRIS-Phosphat-Puff er pH 8,5) bis der A₂₈₀ₘₘ-Wert des Eluats unter 0,5 abgesunken war und weiteres Waschen mit 400 ml 35 mM TRIS-Phosphat-Puffer pH 6,0 bis der A₂₈₀ₘₘ-Wert des Eluats < 0,4 war, wurden ca. 70 - 80 % der Fremdproteine (bezogen auf die optische Dichte der Ausgangslösung bei 280 nm) eluiert. Die Hauptfraktion mit Ancrod wurde mit 85 % Ausbeute in 150 - 200 ml 150 mM TRIS-Phosphat-Puffer pH 6,0 eluiert.

### b. Hauptreinigung

Das Eluat, das das Ancrod enthielt, wurde mittels Ultrafiltration an einer Membran mit einer nominellen Trenngrenze von 10000 Dalton auf 20 ml aufkonzentriert und mit einem 100 mM TRIS-Phosphat-Puffer pH 8,0 umgepuffert. Diese Lösung wurde auf eine Säule aufgetragen, die einen Durchmesser von 1,6 cm hatte und bis zu einer Höhe von 15 cm mit BIORAN-CPG Glas (Firma Schott, Porendurchmesser: 25 - 35 nm, Korngröße: 30 - 60 µm) gefüllt war. Durch Waschen der Säule bei Raumtemperatur mit 300 ml des 100 mM TRIS-Phosphat-Puffers pH 8,0 und mit einer Durchflußgeschwindigkeit von 250 ml pro Stunde wurden ca. 60 % Fremdproteine (bezogen auf die optische Dichte des Auftrags bei 280 nm) von der Säule eluiert.

Zur Elution wurde eine 0,5 M Natriumchlorid-Lösung, die mit 100 mM TRIS-Phosphat auf einen pH-Wert von 8,0 abgepuffert war, eingesetzt. Das Eluat wurde automatisch in ca. 10 ml Fraktionen aufgefangen. Die thrombinähnlichen Enzyme wurden in einem Peak mit anschließendem Tailing-Bereich eluiert. Um die Hauptkomponente (entspricht Ancrod) in einer Form zu erhalten, die höchstens 5 % an thrombinähnlichen Nebenkomponenten enthielt, wurden. beim Übergang vom Hauptpeak zum Tailing-Bereich einzelne Fraktionen sowohl auf ihre spezifische Fibrinogenase-Aktivität als auch auf ihre Zusammensetzung mittels Umkehrphasen-HPLC untersucht. Es wurden nur Fraktionen, die eine spezifische Aktivität von größer 1700 U/OD₂₈₀ₙₘ besaßen und die in HPLC weniger als 10 % an Nebenkomponenten aufwiesen, mit dem Hauptpeak vereinigt (ca. 80 ml). Die Hauptkomponente wurde mit einer Reinheit von 96 % und einer Ausbeute von 72 % von der BIORAN-Säule erhalten.

### c. Feinreinigung

Das Eluat mit der Hauptkomponente Ancrod wurde durch Ultrafiltration an einer YM 10 Membran (Firma Amicon) auf 2 ml aufkonzentriert und das erhaltene Konzentrat auf eine Säule aufgetragen, die einen Durchmesser von 1,6 cm hatte und bis zu einer Höhe von 85 cm mit SEPHARYL S-100 HR gefüllt war. Die Säule war zuvor mit einem Puffer von 100 mM Natriumchlorid und 100 mM Natriumphosphat, der einen pH-Wert von 6,9 aufwies, equilibriert worden. Mit dieser Gelchromatographie wurden restliche Proteasen sowie TRIS von Ancrod abgetrennt. Die Ausbeute betrug bei diesem Schritt ca. 90 %.

### Beispiel 2

### a. Vorreinigung

2,1 g getrocknetes Gift der malaiischen Grubenotter wurden in 50 ml 35 mM TRIS-Phosphat-Puffer pH 8,5 gelöst, unlösliche Zellbestandteile des Giftes abzentrifugiert und die klare, gelbe Lösung auf eine Chromatographie-Säule aufgetragen, welche einen Durchmesser von 1,6 cm aufwies, bis zu einer Höhe von 30 cm mit DEAE-SEPHAROSE-FF (Firma Pharmacia) gefüllt war und mit dem oben genannten Puffer equilibriert war. Durch Waschen der Säule bei Raumtemperatur mit 600 ml Equilibrierungspuffer bis der A₂₈₀-Wert des Eluats < 0,2 war, wurden etwa 70 % der Fremdproteine (bezogen auf die optische Dichte der Ausgangslösung bei 280 nm) eluiert. Die Hauptfraktion wurde mit 90 - 100%iger Ausbeute mit 200 - 250 ml 150 mM TRIS-Phosphat-Puffer pH 6,0 eluiert.

### b. Hauptreinigung

Das Eluat wurde wie in Beispiel 1 auf 20 ml aufkonzentriert und mit einem 50 mM Na-Phosphat Puffer pH 8,5 umgepuffert. Diese Lösung wurde auf die BIORAN-CPG-Glas-Säule (Durchmesser: 1,6 cm, Höhe: 15 cm, Porendurchmesser: 25 - 35 nm, Korngröße: 30 - 60 µm) aufgetragen. Durch Waschen der Säule bei Raumtemperatur mit 300 ml des 50 mM Na-Phosphat-Puffers pH 8,5 und mit einer Durch-. flußgeschwindigkeit von 250 ml pro Stunde wurden ca. 60 % Fremdproteine (bezogen auf die optische Dichte des Auftrags bei 280 nm) von der Säule eluiert.

Zur Elution der thrombinähnlichen Enzyme wurde eine 1 M Natriumchlorid-Lösung, die mit 50 mM Na-Phosphat auf einen pH-Wert von 8,0 abgepuffert worden war, eingesetzt. Mit 150 ml Puffer wurden ca. 80 % der aufgetragenen Einheiten an Enzym eluiert.

### c. Feinreinigung

Das Eluat wurde wie oben auf 20 ml aufkonzentriert und mit einem 50 mM Na-Phosphat-Puffer, der einen pH-Wert von 5,0 aufwies, umgepuffert. Diese Lösung wurde auf eine Säule auf getragen, die ebenfalls einen Durchmesser von 1,6 cm hatte und bis zu einer Höhe von 15 cm mit BIORAN-CPG-Glas gefüllt war, das jedoch einen Porendurchmesser von 90 - 110 nm und eine Korngröße von 30 - 60 µm besaß. Bei pH 5,0 wurden nur basische Proteine sowie die Nebenkomponenten an das BIORAN-Glas gebunden, während die Hauptkomponente Ancrod mit dem Äquilibrierungspuffer in einer Reinheit von annähernd 100 % und mit ca. 80 - 90%iger Ausbeute (bezogen auf die aufgetragenen Einheiten) von der Säule eluiert wurde.

### Beispiel 3

### a, b. Vor- und Hauptreinigung

2,1 g getrocknetes Gift der malaiischen Grubenotter wurden analog Beispiel 2 an DEAE-SEPHAROSE vorfraktioniert, das Eluat analog Beispiel 1 auf 20 ml aufkonzentriert und mit einem 40 mM TRIS-Phosphat-Puffer pH 6,2 umgepuffert. Diese Lösung wurde auf eine Chromatographie-Säule aufgetragen, welche einen Durchmesser von 1,6 cm aufwies, bis zu einer Höhe von 20 cm mit CM-SEPHAROSE-FF (Firma Pharmacia) gefüllt und mit dem oben genannten Puffer equilibriert war. Durch Waschen der Säule bei Raumtemperatur mit 300 ml des Equilibrierungspuffers und einer Durchflußgeschwindigkeit von 250 ml/h wurden ca. 60 % Fremdproteine (bezogen auf die optische Dichte des Auftrags bei 280 nm) von der Säule eluiert.

Zur Elution der thrombinähnlichen Enzyme wurde analog Beispiel 2 eine 1 M Natriumchlorid-Lösung, die mit 50 mM Na-Phosphat auf einen pH-Wert von 8,0 abgepuffert war, eingesetzt. Mit 150 ml Puffer wurden ca. 80 % der aufgetragenen Einheiten an thrombinähnlichen Enzymen eluiert.

### c. Feinreinigung

Die Feinreinigung von Ancrod wurde analog Beispiel 2 an BIORAN-CPG-Glas (Porendurchmesser ca. 100 nm; Korngröße 30 - 60 µm) mit einem 50 mM Phosphat-Puffer pH 5,0 durchgeführt. Ancrod wurde in über 95%iger Reinheit und mit ca. 85%iger Ausbeute (bezogen auf die aufgetragenen Einheiten) von der Säule eluiert.

## Patentansprüche

1. Verfahren zur Reinigung von thrombinähnlichen Proteasen aus Schlangengiften, **dadurch gekennzeichnet, daß** man
a. ein Protease-Rohprodukt einer Vorreinigung durch Affinitätschromatographie oder Chromatographie an einem basischen Ionenaustauscher unterwirft,
b. die so erhaltene Fraktion mit den thrombinähnlichen Enzymen einer Chromatographie an einem schwachen Kationenaustauscher unterwirft oder durch Adsorption an Glas im basischen Bereich trennt und
c. die Hauptkomponente aus Schritt b einer Gelchromatograhie unterwirft oder diese durch Chromatographie an Glas im sauren Bereich reinigt,
wobei jedoch mindestens einer der Schritte b und c eine Trennung durch Chromatographie an Glas beinhaltet.

2. Verfahren gemäß Anspruch 1, **dadurch gekennzeichnet, daß** die Reinigung durch Adsorption in Schritt b an Glas erfolgt und Schritt c mittels Gelchromatographie durchgeführt wird.

3. Verfahren gemäß Anspruch 1, **dadurch gekennzeichnet, daß** die Reinigung durch Chromatographie in den Schritten b und c an Glas erfolgt.

## Claims

1. Process for the purification of thrombin-like proteases from snake venoms, **characterized in that**
a. a protease crude product is subjected to a preliminary purification by affinity chromatography or chromatography on a basic ion exchanger,
b. the resulting fraction containing the thrombin-like enzymes is subjected to chromatography on a weak cation exchanger or separated by adsorption on glass in the basic range, and
c. the main component from step b is subjected to gel chromatography or purified by chromatography on glass in the acidic range,
wherein at least one of steps b and c comprises a separation by chromatography on glass.

2. Process according to Claim 1, **characterized in that** the purification by adsorption in step b takes place on glass and step c is carried out by gel chromatography.

3. Process according to Claim 1, **characterized in that** the purification by chromatography in steps b and c takes place on glass.

## Revendications

1. Procédé pour purifier des protéases analogues à la thrombine issues de venins de serpent, **caractérisé en ce que**
a. on soumet un produit brut protéase à une pré-purification par chromatographie d'affinité ou chromatographie sur un échangeur d'ions basique,
b. on soumet la fraction contenant les enzymes analogues à la thrombine ainsi obtenue à une chromatographie sur un échangeur de cations faible ou on la sépare par adsorption sur du verre dans le domaine basique, et
c. on soumet le composant principal provenant de l'étape b à une chromatographie sur gel ou on le purifie par chromatographie sur du verre dans le domaine acide,
où, toutefois, au moins l'une des étapes b et c contient une séparation par chromatographie sur du verre.

2. Procédé selon la revendication 1 **caractérisé en ce que** la purification par adsorption dans l'étape b se déroule sur du verre et l'étape c est réalisée par chromatographie sur gel.

3. Procédé selon la revendication 1 **caractérisé en ce que** la purification par chromatographie dans les étapes b et c se déroule sur du verre.
